# FASCICULE DE BREVET EUROPEEN

(11) **EP 3 983 005 B1**
(45) Date de publication et mention de la délivrance du brevet: **09.08.2023**
(21) Numéro de dépôt: 20742803.8
(22) Date de dépôt: 09.06.2020
(51) Int. Cl.: A61K 39/00, A61K 39/395, G01N 33/50, A61P 35/00

(54) **COMBINAISON DE MARQUEURS POUR PRÉDIRE LA RÉPONSE À VX-001**
KOMBINATION VON MARKERN ZUR VORHERSAGE DER REAKTION AUF VX-001
COMBINATION OF MARKERS FOR PREDICTING THE RESPONSE TO VX-001

(30) Priorité: 11.06.2019 FR 1906208
(43) Date de publication de la demande: 20.04.2022
(73) Titulaire: Kriptic Pharmaceuticals Limited, Dublin 1 (IE)
(72) Inventeur: KOSMATOPOULOS, Kostantinos (Kostas), 75005 PARIS (FR); MENEZ-JAMET, Jeanne, 92120 MONTROUGE (FR)
(74) Mandataire: Santarelli
(86) Numéro de dépôt international: PCT/FR2020/050975
(87) Numéro de publication internationale: WO 2020/249895

(56) Documents cités:
- CESARE GRIDELLI ET AL: "Clinical activity of a htert (vx-001) cancer vaccine as post-chemotherapy maintenance immunotherapy in patients with stage IV non-small cell lung cancer: final results of a randomised phase 2 clinical trial", BRITISH JOURNAL OF CANCER, 25 mars 2020 (2020-03-25), XP055680259, GB ISSN: 0007-0920, DOI: 10.1038/s41416-020-0785-y
- GEORGOULIAS VASSILIS ET AL: "A multicenter randomized phase IIb efficacy study of Vx-001, a peptide-based cancer vaccine as maintenance treatment in advanced non-small-cell lung cancer: treatment rationale and protocol dynamics", CLINICAL LUNG CANCER JUL 2013,, vol. 14, no. 4, 1 juillet 2013 (2013-07-01), pages 461-465, XP002774759, ISSN: 1938-0690, DOI: 10.1016/J.CLLC.2013.02.001
- Anonymous: "vaxon-biotech > VX-001", , 8 avril 2017 (2017-04-08), XP055680260, Extrait de l'Internet: URL:https://web.archive.org/web/2017040815 5422/http://vaxon-biotech.com/?page_id=122 [extrait le 2020-03-27]
- Anonymous: "Novel Vaccine Shows Association With Survival Based on Smoking History in Patients With Advanced NSCLC", , 18 septembre 2017 (2017-09-18), XP055680262, Extrait de l'Internet: URL:https://www.targetedonc.com/conference /esmo-2017/novel-vaccine-shows-association -with-survival-based-on-smoking-history-in -patients-with-advanced-nsclc [extrait le 2020-03-27]
- KOTSAKIS A ET AL: "Clinical outcome of patients with various advanced cancer types vaccinated with an optimized cryptic human telomerase reverse transcriptase (TERT) peptide: Results of an expanded phase II study", ANNALS OF ONCOLOGY, KLUWER, DORDRECHT, NL, vol. 23, no. 2, 1 février 2012 (2012-02-01), pages 442-449, XP002724182, ISSN: 0923-7534, DOI: 10.1093/ANNONC/MDR396 [extrait le 2011-08-25]
- Anonymous: "Lung cancer: Vaxon announces results of its Vx-001 therapeutic vaccine Phase IIb trial - Andrew Lloyd & Associates", , 1 juin 2017 (2017-06-01), XP055680267, Extrait de l'Internet: URL:https://ala.com/vaxon-announces-result s-of-its-phase-iib-lung-cancer-trial-of-vx -001-a-therapeutic-vaccine-based-on-optimi zed-cryptic-peptides/ [extrait le 2020-03-27]

## Description

### DOMAINE DE L'INVENTION

La présente invention concerne le domaine de l'immunothérapie anticancéreuse, plus particulièrement le domaine de la vaccination antitumorale.

### ARRIERE-PLAN TECHNOLOGIQUE

L'état général des patients atteints de cancer est évalué par des analyses sanguines de routine, incluant le taux de LDH (lactate déshydrogénase), de gGT (gamma Glutamine Transférase), d'ALP (Alcaline Phosphatase), marqueurs de la fonction hépatique et d'inflammation, et/ou une numération sanguine permettant de déterminer le nombre absolu et le rapport entre les neutrophiles et les lymphocytes ainsi que le taux de plaquettes.

Ces constantes sont connues comme des marqueurs pronostics chez des patients atteints de cancers, les patients présentant un état général dégradé ayant un moins bon pronostic vital. Il a aussi été montré que cela pouvait influer la réponse à certains traitements notamment les Immune Checkpoint Inhibiteur, molécules mises au point ces dernières années qui ont révolutionné la prise en charge de certains cancers (Ferrucci et al., Annals Oncol. 2016).

L'immunothérapie anticancéreuse est destinée à stimuler la reconnaissance par les lymphocytes T cytotoxiques (CTL) de peptides dérivés d'antigènes tumoraux qui sont présentés à la surface de la cellule tumorale par des molécules HLA (antigènes des leucocytes humains) de classe I (HLA-I). Les peptides ciblés par les CTL peuvent être dominants ou cryptiques en fonction de leur affinité pour les molécules HLA

Les antigènes associés aux tumeurs (AAT) sont fréquemment exprimés à la fois par les cellules tumorales et les tissus normaux, contrairement aux néo-antigènes qui sont spécifiques à la tumeur et le plus souvent spécifiques au patient. Les AAT présentent l'avantage d'être exprimés par de très nombreuses tumeurs contrairement aux néo-antigènes, mais ils ont l'inconvénient d'être soumis aux mécanismes de tolérance qui empêchent l'auto-immunité, c'est à dire la reconnaissance d'antigènes du soi par le système immunitaire d'un individu.

La télomérase transcriptase inverse (TERT), enzyme impliquée dans l'immortalisation des cellules tumorales, est un antigène de type TAA exprimé par de nombreuses tumeurs (85% des tumeurs). Pour contourner le problème de la tolérance du système immunitaire vis-à-vis des AAT, tout en ciblant un antigène largement exprimé par les tumeurs, les inventeurs ont proposé un vaccin (Vx-001) ciblant un peptide cryptique de la TERT, c'est-à-dire un peptide de la TERT qui présente une faible affinité pour la molécule HLA-A*0201 (molécule du CMH-I la plus fréquemment exprimé chez l'homme) et forme des complexes peptide / HLA-I instables (Tourdot S. et al., 2000, Menez-Jamet J. et al., 2016). Étant donné la forte corrélation entre l'affinité pour HLA-I et l'immunogénicité, ce peptide cryptique n'est naturellement pas immunogène. Pour l'utiliser comme vaccin contre le cancer, ce peptide cryptique a été optimisé pour renforcer son immunogénicité.

Le Vx-001 est donc un vaccin thérapeutique antitumoral composé de deux peptides de neuf acides aminés : le peptide cryptique natif TERT572 (RLFFYRKSV, SEQ ID N ° 1), exprimé par les cellules tumorales, et son variant optimisé TERT572Y (YLFFYRKSV, SEQ ID N ° 2). Ces deux peptides sont administrés séparément, chacun avec l'adjuvant Montanide ISA51^{®}VG (un mélange d'une huile minérale hautement purifiée (Drakeol 6VR) et d'un surfactant (Mannide monooléate)). Le TERT572Y, peptide optimisé immunogène, est utilisé pour les deux premières vaccinations, afin de déclencher une réponse immunitaire importante. Le peptide natif TERT572 est administré dans les étapes vaccinales suivantes, afin de sélectionner, parmi tous les lymphocytes T stimulés par TERT572Y, ceux qui possèdent la plus haute spécificité pour le TERT572 natif, qui est présenté à la surface des cellules tumorales associées au HLA-A*0201.

Le Vx-001 a été récemment testé chez des patients atteints de NSCLC (cancer du poumon non à petites cellules) métastatique, ou récurrent de stade I-III, dans une étude clinique de phase Ilb randomisée (étude Vx-001-201). Le protocole vaccinal dans cette étude comprenait 6 vaccinations à 3 semaines d'intervalles (2 vaccinations avec TERT572Y, puis 4 vaccinations avec TERT572), puis des rappels toutes les 12 semaines (avec TERT572) jusqu'à la progression de la maladie (Georgoulias et al., Clin Lung Cancer. 2013).

Les patients inclus exprimaient HLA-A*0201 et avaient une tumeur exprimant TERT. L'objectif principal de l'étude était le suivie de la survie globale. Les résultats de cette étude n'ont pas été globalement statistiquement significatifs. Toutefois, le Vx-001 a montré une efficacité significative chez certains patients. Les inventeurs ont donc étudié si certaines constantes sanguines pouvaient être liées à une réponse ou une absence de réponse clinique au vaccin. Cette étude a conduit à une stratification des patients, avec identification de catégories de patients pour lesquels la vaccination par Vx-001 s'est avérée statistiquement bénéfique.

### RESUME DE L'INVENTION

Les inventeurs ont mis en évidence que le vaccin antitumoral Vx-001 apporte un bénéfice statistiquement significatif aux patients présentant un taux normal de gGT et/ou un taux normal de LDH, les patients ayant des taux de gGT et de LDH normaux répondant particulièrement bien au traitement. Une stratification plus fine a montré que les patients masculins ayant une tumeur non-épidermoïde répondaient particulièrement bien au traitement.

La présente invention porte donc sur l'utilisation du Vx-001 dans une méthode de traitement d'une tumeur exprimant la télomérase transcriptase inverse (TERT), chez un patient HLA-A*0201 présentant un taux normal de gamma Glutamine Transférase (gGT) et/ou un taux normal de lactate déshydrogénase (LDH), de préférence chez un patient masculin ayant une tumeur non-épidermoïde.

La présente invention porte également sur une méthode pour déterminer *in vitro* si un patient HLA-A*0201 atteint d'une tumeur exprimant la TERT est susceptible de répondre favorablement à une vaccination anticancéreuse avec le Vx-001, comprenant la mesure des taux de gGT et de LDH du patient, qui est susceptible de répondre favorablement à la vaccination par Vx-001 si au moins un de ces taux est normal.

### LEGENDES DES FIGURES

**Figure 1** : **OS et TTF chez les patients ayant un taux sérique de LDH normal.** Courbe épaisse grise : patients ayant reçu le Vx-001 ; courbe noire en traits fins : patients ayant reçu un placebo.
**Figure 2** : **OS et TTF chez les patients ayant un taux sérique normal de gGT.** Courbe épaisse grise : patients ayant reçu le Vx-001 ; courbe noire en traits fins : patients ayant reçu un placebo.
**Figure 3** : **Réponse clinique au Vx-001 chez les patients ayant un taux sérique d'ALP normal.** Courbe épaisse grise : patients ayant reçu le Vx-001 ; courbe noire en traits fins : patients ayant reçu un placebo.
**Figure 4** **: Réponse clinique au Vx-001 et nombre absolu de neutrophiles.** Courbe épaisse grise : patients ayant reçu le Vx-001 ; courbe noire en traits fins : patients ayant reçu un placebo.
**Figure 5** : **Réponse clinique au Vx-001 et rapport entre neutrophiles et lymphocytes.** Courbe épaisse grise : patients ayant reçu le Vx-001 ; courbe noire en traits fins : patients ayant reçu un placebo.
**Figure 6****. Réponse clinique au Vx-001 et nombre de plaquettes.** Courbe épaisse grise : patients ayant reçu le Vx-001 ; courbe noire en traits fins : patients ayant reçu un placebo.
**Figure 7** : **OS et TTF chez les patients ayant des taux sériques normaux de LDH et de gGT.** Courbe épaisse grise : patients ayant reçu le Vx-001 ; courbe noire en traits fins : patients ayant reçu un placebo.
**Figure 8** : **Analyse de la survie des sous-groupes de patients ayant des taux sériques normaux de LDH et de gGT.** NSQ : tumeur non-épidermoïde. SQ : tumeur épidermoïde. OR : réponse objective à la chimiothérapie. SD : maladie stable. ECOG : indice fonctionnel *"Eastern Cooperative Oncology Group".*
**Figure 9** : **Analyse du TTF (Time to Treatment Failure), des sous-groupes de patients ayant des taux sériques normaux de LDH et de gGT.** NSQ : tumeur non-épidermoïde. SQ : tumeur épidermoïde. OR : réponse objective à la chimiothérapie. SD : maladie stable. ECOG : indice fonctionnel "Eastern Cooperative Oncology Group".
**Figure 10** **: Survie des patients en fonction de la combinaison de marqueurs étudiée.** Courbe épaisse grise : patients ayant reçu le Vx-001 ; courbe noire en traits fins : patients ayant reçu un placebo.
**Figure 11** : **Survie des patients en fonction de l'immunogénicité de la tumeur définie par l'infiltration par des TIL (Tumor Infiltrating Lyphocytes) et l'expression de PD-L1.** Courbe grise : patients ayant reçu le Vx-001 ; courbe noire: patients ayant reçu un placebo.
**Figure 12** : **TTF des patients en fonction de l'immunogénicité de la tumeur définie par l'infiltration par des TIL (Tumor Infiltrating Lymphocytes) et l'expression de PD-L1.** Courbe grise : patients ayant reçu le Vx-001 ; courbe noire: patients ayant reçu un placebo.
**Figure 13** : **Survie des patients dont la tumeur est non-immunogénique ou immunogénique en fonction des marqueurs étudiés.** LDH/gGT UNL = LDH/gGT inférieurs aux limites supérieures de normalité.
**Figure 14** **: TTF des patients dont la tumeur est non-immunogénique ou immunogénique en fonction des marqueurs étudiés.** LDH/gGT UNL = LDH/gGT inférieurs aux limites supérieures de normalité.

### DESCRIPTION DETAILLEE

La présente invention est basée sur l'identification de catégories de patients pour lesquels les résultats de l'étude Vx-001-201 ont montré que la vaccination par Vx-001 avait été significativement bénéfique.

La présente invention concerne, pour chacune de ces catégories de patients (énoncées ci-dessous), l'utilisation de Vx-001 pour traiter une tumeur exprimant la TERT.

La présente invention concerne également, pour chacune de ces catégories de patients, l'utilisation du peptide TERT572Y de SEQ ID No 2, pour induire une réponse CTL contre l'épitope cryptique TERT572 de séquence SEQ ID No : 1.

L'invention concerne aussi, pour chacune de ces catégories de patients, l'utilisation du peptide TERT572 de séquence SEQ ID No : 1 pour maintenir une réponse CTL induite par le peptide TERT572Y de SEQ ID No 2 préalablement administré au patient.

Comme illustré dans la partie expérimentale ci-dessous, une première catégorie de patients est constituée des patients HLA-A*0201 atteints d'une tumeur exprimant la TERT et présentant un taux normal de lactate déshydrogénase (LDH).

Une autre catégorie de patients susceptibles de répondre favorablement à la vaccination par Vx-001 est constituée des patients HLA-A*0201 atteints d'une tumeur exprimant la TERT et présentant un taux normal de gamma Glutamine Transférase (gGT).

Les patients HLA-A*0201 atteints d'une tumeur exprimant la TERT et présentant à la fois un taux normal de gGT et un taux normal de LDH constituent une catégorie de patients particulièrement susceptibles de répondre favorablement à la vaccination par le Vx-001.

Par « taux normal de gGT » et « taux normal de LDH », on entend des taux inférieurs aux limites supérieures de normalité (*Upper normal limits* ; UNL) définis par chaque laboratoire en fonction de la technique et des réactifs utilisés dans ce laboratoire. L'identification du caractère normal ou non d'un taux ne présente aucune difficulté pour l'homme du métier.

La présente invention porte donc sur l'utilisation du Vx-001, pour traiter une tumeur exprimant la TERT, chez un patient HLA-A*0201 présentant un taux normal de gGT et/ou un taux normal de LDH.

Par « traiter », on entend ici au moins ralentir la progression de la tumeur et/ou prolonger la survie des malades.

Selon une mise en oeuvre particulière de l'invention, le Vx-001 est administré à un patient présentant un taux normal de gGT et/ou un taux normal de LDH après avoir reçu au moins une chimiothérapie. Le Vx-001 est alors administré à la suite d'une chimiothérapie de première ligne.

Selon une autre mise en oeuvre particulière de l'invention, le Vx-001 est administré à un patient atteint d'un cancer du poumon non à petites cellules (NSCLC), par exemple un NSCLC métastatique ou récurrent, ledit patient présentant un taux normal de gGT et/ou un taux normal de LDH.

Les inventeurs ont également démontré que parmi les patients présentant un taux normal de gGT et/ou un taux normal de LDH, les hommes répondaient statistiquement mieux à la vaccination par Vx-001 que les femmes.

Selon une autre mise en oeuvre particulière de l'invention, le Vx-001 est donc administré à un patient masculin présentant un taux normal de gGT et/ou un taux normal de LDH.

De même, les résultats de l'étude Vx-001-201 montrent que le Vx-001 est statistiquement plus efficace pour traiter les tumeurs non-épidermoïdes que les tumeurs épidermoïdes.

Selon une autre mise en oeuvre particulière de l'invention, le Vx-001 est donc administré à un patient présentant un taux normal de gGT et/ou un taux normal de LDH souffrant d'une tumeur non-épidermoïde.

Selon une autre mise en oeuvre particulière de l'invention, le Vx-001 est administré à un patient de moins de 65 ans présentant un taux normal de gGT et/ou un taux normal de LDH.

Selon une autre mise en oeuvre particulière de l'invention, le Vx-001 est administré à un patient présentant un taux normal de gGT et/ou un taux normal de LDH et dont le cancer ne progresse plus après une chimiothérapie, par exemple après une chimiothérapie de première ligne.

Selon une autre mise en oeuvre particulière de l'invention, le Vx-001 est administré à un patient présentant un taux normal de gGT et/ou un taux normal de LDH et ayant un score ECOG 0.

Les résultats de l'étude Vx-001-201 montrent également que les taux normaux de LDH et de gGT sériques corrèlent avec une plus longue survie et un plus long TTF chez des patients qui ont des tumeurs non-immunogéniques. Par « tumeur non-immunogénique », on entend ici une tumeur qui n'exprime pas la molécule PD-L1 (c'est-à-dire, si moins de 1% des cellules tumorales expriment PD-L1 : Tumor Proportional Score <1%) et/ou qui n'est pas infiltrée par des lymphocytes (c'est-à-dire, lorsque les lymphocytes infiltrant la tumeur, ou TIL, sont totalement absents ou très peu nombreux).

Selon une autre mise en oeuvre particulière de l'invention, le Vx-001 est donc administré à un patient présentant un taux normal de gGT et/ou un taux normal de LDH, et dont la tumeur n'exprime pas la molécule PD-L1 et/ou n'est pas infiltrée par des lymphocytes.

La présente invention porte également sur une méthode pour déterminer *in vitro* si un patient HLA-A*0201 atteint d'une tumeur exprimant la TERT est susceptible de répondre favorablement à une vaccination thérapeutique anticancéreuse avec le Vx-001, comprenant une étape de mesure, à partir d'un échantillon biologique dudit patient, du taux de gGT et du taux de LDH dudit patient ; selon cette méthode, le patient est considéré comme susceptible de répondre favorablement à la vaccination anticancéreuse si au moins un de ces taux est normal, et a fortiori si les taux de gGT et de LDH du patient sont tous les deux normaux.

Selon la méthode de l'invention, on considère que la probabilité que le patient réponde favorablement à la vaccination anticancéreuse par Vx-001 est encore plus élevée si la tumeur est non-immunogénique, c'est-à-dire si elle n'exprime pas la molécule PD-L1 et/ou n'est pas infiltrée par des lymphocytes.

Selon la méthode de l'invention, on considère que la probabilité que le patient réponde favorablement à la vaccination thérapeutique anticancéreuse par Vx-001 est encore plus élevée si le patient présente en outre une ou plusieurs des caractéristiques suivantes :
(i) il est de sexe masculin
(ii) il a une tumeur non-épidermoïde
(iii) il a moins de 65 ans
(iv) il a une tumeur qui ne progresse plus après une première ligne de chimiothérapie
(v) il a un ECOG=0.

Bien entendu, les différents modes de mise en oeuvre de l'invention ne sont pas exclusifs les uns des autres, et les catégories de patients possédant plusieurs des critères énoncés ci-dessus sont d'autant plus susceptibles d'être de bons répondeurs à la vaccination par Vx-001.

La présente invention est davantage illustrée dans la partie expérimentale ci-dessous, qui n'en limite pas la portée.

### EXEMPLES

Les analyses sanguines ont été réalisées au cours du processus de recrutement des patients dans l'étude clinique, après le traitement par chimiothérapie. Chaque laboratoire appliquant ses propres tests et ses propres limites de normalité (UNL ou *"Upper Normal Limit"*)*,* les données ont été collectées dans le dossier médical des patients et classées en fonction des résultats obtenus par rapport à la limite de normalité appliquée par le laboratoire (tableau 1). Les éléments étudiés sont le taux de LDH, de gGT, d'ALP, le nombre absolu de neutrophiles, le nombre absolu de plaquettes ainsi que le rapport entre les neutrophiles et les lymphocytes.

L'efficacité du produit d'investigation (Vx-001) dans cet essai clinique a été évaluée en comparant deux critères entre les patients qui ont reçu le produit d'investigation et ceux qui ont reçu le placebo :
- la survie du patient ou "*overall survival"* (noté OS)
- la période pendant laquelle la maladie ne progresse plus et le malade ne reçoit aucun autre traitement que le produit d'investigation (ou le placebo), appelé *"Time to Treatment Failure",* noté TTF.

**Tableau 1 : Mesure du taux sérique de Lactate déshydrogénase (LDH), gamma Glutamine Transférase (gGT), Alcaline Phosphatase (ALP), du nombre absolu de neutrophiles (ANC), du rapport des neutrophiles/lymphocytes (NIL) et de plaquettes (Plaq). Les patients inclus dans l'étude Vx001-201 ont été classés en plusieurs sous-groupes : L pour les patients ayant un taux normal pour le marqueur considéré, en accord avec les standards du laboratoire qui a effectué le dosage, et H pour les patients ayant un taux supérieur à la limite normale établie par chaque laboratoire ; N=Non ; O= Oui ; ND=non mesuré.**

| Patient | LDH (UNL) | gGT (UNL) | ANC> 6000 | N/L>3 | Plaq (UNL) | ALP > UNL | Patient | LDH (UNL) | gGT (UNL) | ANC> 6000 | N/L>3 | ALP > UNL | Plaq (UNL) |
|---|---|---|---|---|---|---|---|---|---|---|---|---|---|
| 01-02-004 | L | L | N | O | N | N | 04-07-021 | ND | ND | N | N | N | N |
| 01-03-007 | L | L | N | N | N | N | 04-07-029 | H | L | N | N | N | N |
| 01-03-012 | L | H | N | N | N | N | 04-09-005 | L | L | O | O | N | N |
| 01-03-018 | L | L | N | N | N | N | 04-10-007 | ND | ND | O | O | N | O |
| 01-03-023 | L | H | N | N | O | N | 04-10-008 | H | ND | N | O | N | N |
| 01-04-011 | L | H | N | O | O | O | 04-10-014 | L | L | N | O | N | N |
| 01-05-001 | L | L | N | N | N | N | 04-10-024 | L | L | O | O | N | O |
| 01-05-011 | L | ND | N | N | N | N | 04-10-025 | ND | L | N | O | N | N |
| 01-06-006 | L | L | N | N | N | N | 04-10-031 | H | L | N | N | N | O |
| 01-07-001 | H | H | N | O | O | O | 04-10-034 | ND | L | N | O | N | N |
| 01-08-002 | L | L | N | O | N | N | 04-12-001 | L | L | N | N | N | N |
| 01-08-003 | L | H | O | O | N | N | 04-12-002 | ND | L | N | O | N | N |
| 01-08-005 | L | L | N | N | N | N | 04-12-009 | L | L | N | N | N | N |
| 01-09-002 | L | L | N | N | N | N | 04-13-003 | L | L | N | N | N | N |
| 01-09-006 | H | L | N | N | N | N | 04-13-004 | L | L | N | N | N | N |
| 01-10-002 | L | L | N | N | N | N | 04-13-011 | ND | L | N | O | N | O |
| 01-10-004 | H | L | N | O | N | N | 04-13-015 | L | L | N | O | N | O |
| 02-03-001 | ND | L | N | N | O | N | 05-01-013 | L | L | N | O | N | N |
| 02-05-002 | ND | L | N | N | N | N | 05-03-003 | L | L | N | N | O | N |
| 02-06-002 | L | L | N | N | N | N | 05-04-001 | H | L | N | O | N | N |
| 02-06-005 | H | H | N | N | N | N | 05-04-010 | L | L | N | N | N | N |
| 02-07-004 | ND | L | N | N | N | N | 05-04-012 | L | H | N | O | O | O |
| 02-07-006 | L | L | N | O | N | N | 05-04-014 | L | L | N | N | N | N |
| 02-07-007 | ND | L | N | N | O | ND | 05-06-005 | L | L | O | O | N | N |
| 03-01-005 | H | L | N | N | N | N | 05-07-006 | ND | ND | N | N | N | N |
| 03-01-008 | H | H | N | N | N | N | 05-07-009 | L | L | N | O | N | N |
| 03-02-001 | L | H | O | O | N | N | 05-07-012 | L | L | N | O | N | N |
| 03-02-033 | L | H | N | O | N | O | 05-07-015 | L | L | N | O | N | N |
| 03-02-042 | L | L | N | N | O | N | 05-07-016 | H | L | N | N | N | N |
| 03-02-044 | L | L | N | O | N | N | 05-08-002 | H | L | N | N | O | N |
| 03-02-056 | L | L | N | N | O | N | 05-08-027 | L | L | N | N | N | N |
| 03-02-060 | ND | L | N | O | N | N | 05-09-004 | L | L | N | N | ND | O |
| 03-02-070 | L | L | N | N | N | N | 05-09-006 | H | H | O | N | ND | N |
| 03-02-100 | L | L | O | O | N | N | 05-09-008 | L | L | N | O | N | N |
| 03-03-001 | L | L | N | N | O | N | 05-10-008 | H | L | N | O | N | N |
| 03-03-012 | L | H | N | N | N | N | 05-11-001 | ND | L | N | N | N | N |
| 03-03-015 | L | L | N | N | O | ND | 05-11-007 | L | L | N | N | O | N |
| 03-03-019 | L | H | O | O | N | N | 05-13-005 | L | L | N | O | N | N |
| 03-03-020 | L | L | N | N | N | ND | 05-13-006 | L | L | O | O | N | N |
| 03-03-023 | L | H | N | O | O | ND | 06-01-002 | L | ND | O | O | O | ND |
| 03-03-025 | L | L | N | N | N | ND | 07-02-004 | L | H | N | N | N | N |
| 03-03-028 | H | H | N | N | N | ND | 07-02-013 | L | ND | O | N | O | N |
| 03-03-031 | L | L | N | N | N | N | 07-03-002 | ND | ND | N | O | N | N |
| 03-03-035 | L | L | O | N | N | N | 07-03-004 | L | L | O | O | N | N |
| 03-03-049 | L | L | N | N | N | ND | 07-03-009 | H | L | N | O | O | O |
| 03-03-058 | L | L | O | N | N | N | 07-03-011 | H | L | O | N | O | N |
| 03-03-062 | L | L | N | N | O | N | 07-03-013 | L | L | N | N | O | O |
| 03-03-065 | L | H | O | O | N | N | 07-03-015 | L | H | O | O | O | O |
| 03-03-086 | L | L | N | O | O | O | 07-03-016 | L | ND | N | N | N | N |
| 03-04-001 | H | L | N | O | N | O | 07-03-019 | H | L | N | N | N | O |
| 03-04-014 | L | L | N | N | O | O | 07-03-021 | L | ND | N | O | N | N |
| 03-04-025 | H | L | O | O | N | N | 07-03-022 | H | ND | N | N | N | N |
| 03-04-040 | L | L | N | N | N | N | 07-03-030 | L | L | N | N | N | N |
| 03-04-042 | L | L | O | N | N | N | 07-03-032 | L | L | O | O | N | N |
| 03-05-006 | H | L | N | N | N | O | 07-04-006 | L | ND | N | O | N | N |
| 03-05-007 | ND | H | N | N | N | N | 07-04-010 | L | L | N | N | N | N |
| 03-06-005 | L | L | N | N | N | N | 07-04-011 | L | ND | N | N | N | N |
| 03-06-009 | L | L | N | N | N | N | 07-04-020 | L | L | N | N | N | N |
| 03-06-011 | H | H | N | N | N | N | 07-04-022 | ND | L | N | N | N | N |
| 03-06-012 | L | L | N | N | N | N | 07-04-023 | L | L | N | N | N | N |
| 03-06-016 | L | L | N | N | O | N | 07-05-001 | L | L | N | N | N | N |
| 03-07-001 | ND | H | N | N | N | ND | 07-06-004 | L | L | N | N | N | O |
| 03-07-007 | H | L | O | O | O | N | 07-06-006 | L | H | N | O | N | N |
| 03-07-008 | L | L | O | O | N | N | 07-06-016 | L | L | N | N | N | N |
| 03-07-013 | L | L | N | O | N | O | 07-06-018 | H | H | N | N | N | N |
| 03-07-021 | ND | ND | N | N | N | N | 07-06-020 | L | H | N | N | N | O |
| 03-07-028 | H | L | N | N | N | N | 07-06-024 | L | H | N | N | O | O |
| 03-07-030 | L | L | N | O | N | N | 07-07-011 | L | L | N | N | N | N |
| 03-07-040 | H | L | O | O | N | N | 07-08-001 | L | L | N | N | N | N |
| 03-07-060 | L | L | N | O | N | N | 07-09-001 | L | L | N | N | O | N |
| 03-07-066 | L | L | N | N | N | N | 07-10-002 | L | H | N | N | O | N |
| 03-07-071 | L | L | N | N | N | N | 07-10-005 | L | L | N | N | N | N |
| 03-08-013 | H | H | N | O | N | N | 07-10-012 | L | L | N | N | N | N |
| 03-08-014 | L | L | N | N | N | N | 07-10-017 | L | L | N | O | N | N |
| 03-10-001 | L | L | N | N | N | N | 08-01-022 | L | L | N | N | N | N |
| 04-01-010 | L | L | N | N | N | N | 08-01-031 | L | H | N | N | N | N |
| 04-01-023 | L | L | N | N | N | N | 08-01-043 | L | L | N | O | N | N |
| 04-01-057 | L | H | N | N | N | O | 08-01-046 | L | L | N | N | N | N |
| 04-02-002 | ND | ND | N | O | N | N | 08-01-050 | H | L | N | O | N | N |
| 04-02-004 | L | L | N | N | N | N | 08-01-051 | H | L | N | N | N | N |
| 04-03-008 | L | L | O | O | N | N | 08-01-056 | H | H | N | O | N | N |
| 04-03-010 | H | L | N | N | N | N | 08-01-059 | L | L | N | N | N | N |
| 04-03-011 | L | L | N | N | N | N | 08-01-060 | H | H | N | O | O | N |
| 04-04-002 | L | L | N | O | N | O | 08-01-061 | H | L | N | N | N | N |
| 04-05-002 | L | L | N | O | N | N | 08-01-064 | L | L | N | O | N | N |
| 04-05-006 | H | L | O | O | N | N | 08-01-069 | H | L | N | N | N | N |
| 04-05-026 | L | L | N | N | N | N | 08-01-070 | H | L | N | N | N | N |
| 04-05-036 | L | H | O | N | N | N | 08-01-071 | H | L | N | N | N | N |
| 04-05-046 | ND | L | N | O | N | N | 08-01-072 | L | L | N | O | N | N |
| 04-06-001 | L | H | N | N | N | N | 08-01-073 | L | H | N | N | N | O |
| 04-06-002 | H | L | N | N | N | N | 08-02-006 | L | L | N | N | N | N |
| 04-07-007 | H | L | N | N | N | N | 08-02-011 | L | L | N | O | N | N |
| 04-07-012 | H | L | N | N | N | N | 08-02-016 | H | L | N | N | N | N |
| 04-07-013 | L | L | N | N | N | N | 08-02-017 | H | L | N | N | O | N |
| 04-07-017 | L | L | N | O | O | N | 08-03-008 | L | H | N | N | N | N |

### 1. Réponse clinique au Vx-001 et taux de LDH

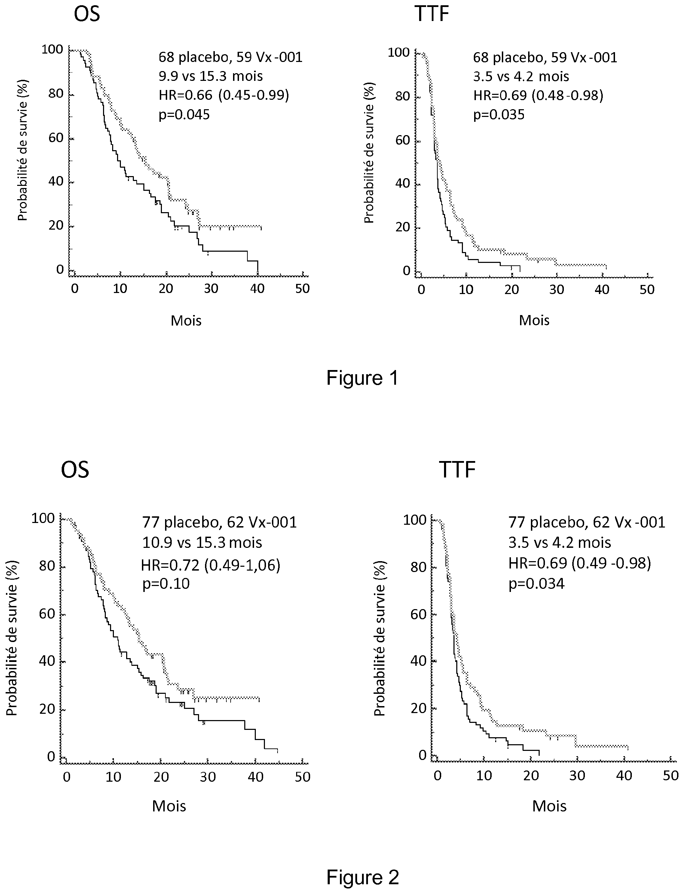

Parmi les 190 patients inclus dans l'étude Vx-001-201, 127 patients avaient un taux sérique de LDH normal. Dans cette population, la vaccination avec le Vx-001 a permis d'augmenter significativement l'OS et le TTF par rapport aux patients du groupe placebo (Figure 1).

### 2. Réponse clinique au Vx-001 et taux de gGT

Parmi les 190 patients inclus dans l'étude Vx-001-201, 139 patients avaient un taux sérique de gGT normal. Dans cette population, la vaccination avec le Vx-001 a permis d'augmenter l'OS par rapport aux patients du groupe placebo mais pas de manière significative (p=0.10). En revanche, le TTF a augmenté significativement (Figure 2).

### 3. Réponse clinique au Vx-001 et alcaline phosphatase (ALP)

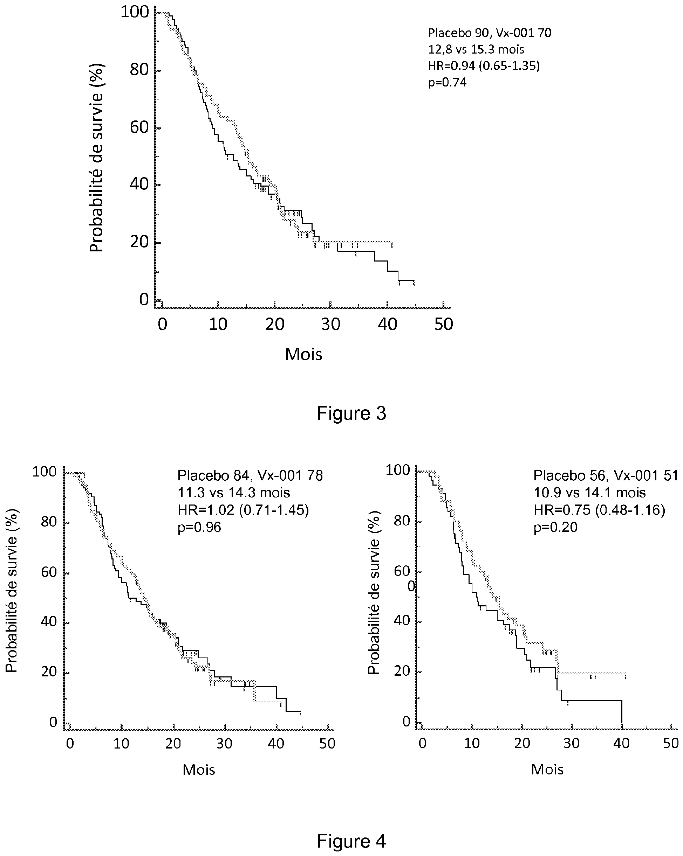

Parmi les 190 patients inclus dans l'étude Vx-001-201, 160 patients avaient un taux sérique d'ALP normal. Dans cette population, la vaccination avec le Vx-001 n'a pas provoqué d'augmentation de l'OS par rapport aux patients du groupe placebo (p=0.74, Figure 3). Il n'y a eu aucun impact du taux d'ALP des patients sur leur capacité à répondre au Vx-001. Les résultats sur le TTF sont identiques.

### 4. Réponse clinique au Vx-001 et numération sanguine

La numération sanguine est couramment utilisée pour évaluer l'état inflammatoire d'un patient. Ainsi, le nombre absolu de neutrophiles, mais aussi le rapport entre le nombre de neutrophiles et de lymphocytes ainsi que le nombre de plaquettes sont caractéristiques de l'état de santé général d'un patient.

### a. Réponse clinique au Vx-001 et nombre absolu de neutrophiles

Le nombre absolu de neutrophiles est un marqueur d'inflammation couramment utilisé. Il a notamment été montré qu'un nombre de neutrophiles élevé était associé à une faible réponse au traitement avec les inhibiteurs de points de contrôles (*"*Immune Checkpoint Inhibitors", ou ICI) (Ferrucci et al., Annals Oncol 2016)

Dans le cas du traitement avec le Vx-001, aucun impact du nombre de neutrophiles n'a été observé. Aucune différence de survie entre les patients du groupe placebo et ceux ayant reçu le traitement n'a été observée, ni chez les patients ayant un taux élevé de neutrophiles, ni chez ceux chez lesquels il était normal (Figure 4).

### b. Réponse clinique au Vx-001 et rapport entre neutrophiles et lymphocytes

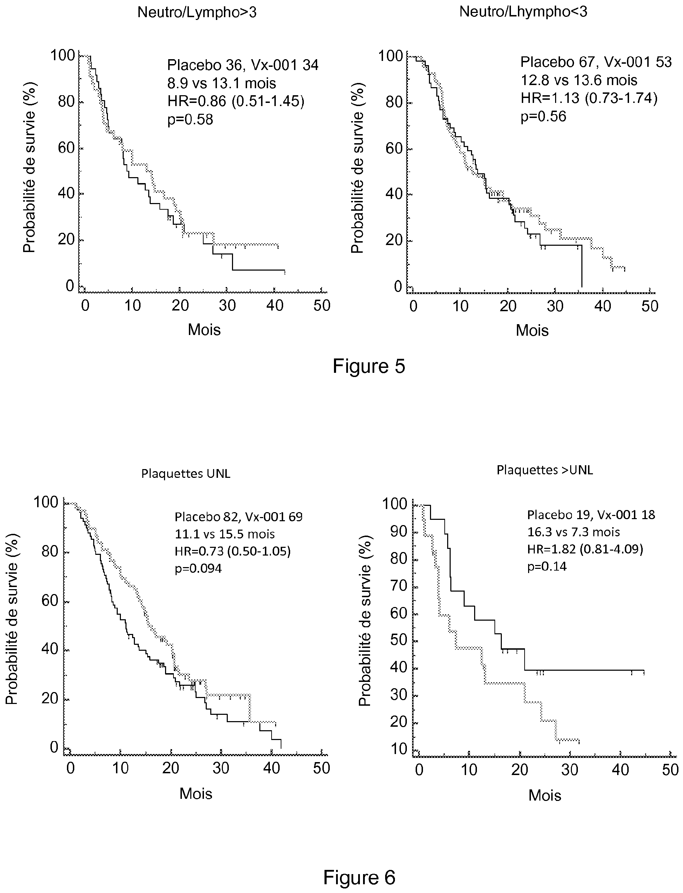

Le même type de résultat a été observé avec le rapport entre le nombre de neutrophiles et de lymphocytes. Aucune différence de survie entre les patients du groupe placebo et ceux ayant reçu le traitement n'a été observée, ni chez les patients ayant un rapport entre neutrophiles et lymphocytes inférieur à 3, rapport couramment utilisé comme limite dans la littérature (Mezquita et al., 2018 ; Ferrucci et al., 2016), ni chez ceux chez lesquels ce taux est supérieur à 3 (Figure 5).

### c. Réponse clinique au Vx-001 et plaquettes

Enfin, le nombre de plaquettes n'a pas d'impact. Aucune différence de survie entre les patients du groupe placébo et ceux ayant reçu le traitement n'a été observée, ni chez les patients ayant un taux élevé de plaquettes, ni chez ceux chez lesquels il était normal (UNL : *"UpperNormal Limit",* ou Limite normale supérieure) (Figure 6).

### 5. Facteurs combinés

### a. gGT et LDH

L'efficacité clinique du Vx-001 a ensuite été évaluée en combinant les facteurs, notamment ceux qui avaient montré un impact sur la survie et le TTF des malades.

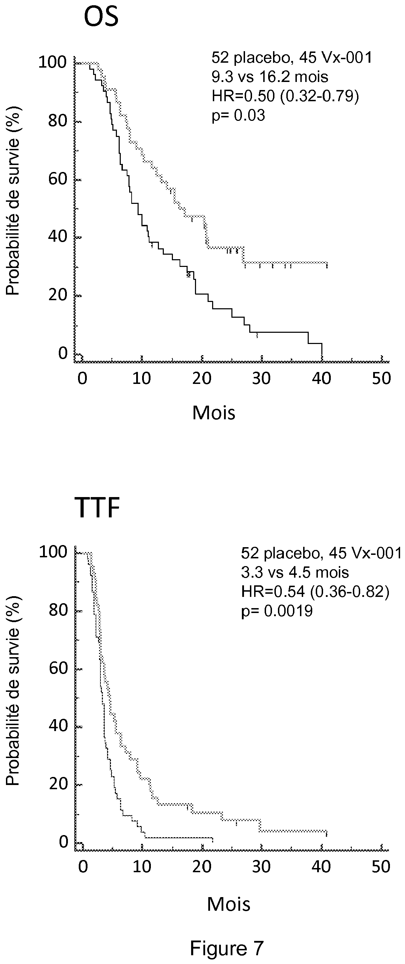

Chez les 97 patients ayant à la fois un taux de LDH et de gGT normal, le traitement avec le Vx-001 a très significativement augmenté la survie et le TTF : p=0.003 et p=0.0019, respectivement (Figure 7).

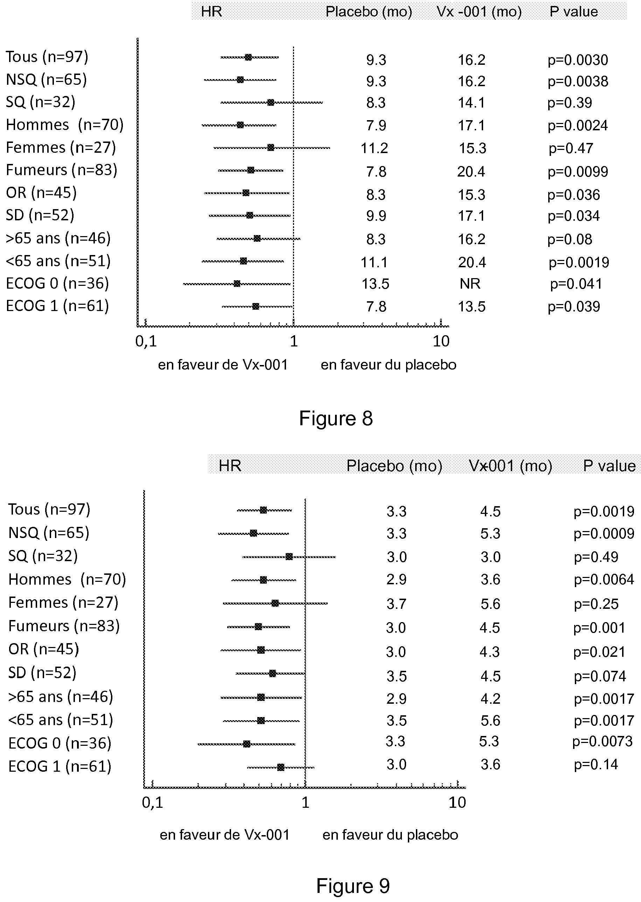

L'analyse plus détaillée de la survie des sous-groupes de cette population de patients avec des taux sériques de LDH et gGT normaux, montre que le Vx-001 a un effet très significatif chez les patients atteints de cancer non-épidermoïdes *("non-squamous"* ou NSQ, p=0.0038), les hommes (p=0.0024), les fumeurs (p=0.0099), les patients de moins de 65 ans (p=0.0019), les patients qui ont intégré l'étude avec une réponse objective à la chimiothérapie (OR, p=0.036) ou en stabilisation (SD, p=0.034), avec un ECOG 0 (0.041) ou un ECOG 1 (p=0.039). En revanche et de façon étonnante, le Vx-001 n'augmente pas significativement la survie des patients dont le cancer a une histologie épidermoïde (SQ, p=0.39), les femmes (p=0.27) et les patients de plus de 65 ans (p=0.08) (Figure 8).

De même, l'analyse du TTF des sous-groupes de cette population de patients avec des taux sériques de LDH et gGT normaux, confirme que le Vx-001 a un effet très important chez les patients atteints de cancer non-épidermoïde (NSQ, p=0.0009), les hommes (p=0.0064), les fumeurs (p=0.001), les patients de moins de 65 ans (p=0.0017), les patients qui ont intégrés l'étude avec une réponse objective à la chimiothérapie (OR, p=0.021), avec un ECOG 0 (0.021), mais également chez les plus de 65 ans (p=0.0017).

En revanche, le Vx-001 n'augmente pas significativement le TTF des patients dont le cancer a une histologie épidermoïde (SQ, p=0.49), les femmes (p=0.25), les patients qui sont entrés dans l'étude avec une maladie stabilisée suite à la chimiothérapie (SD, p=0.074) ou un ECOG 1 (p=0.14) (Figure 9).

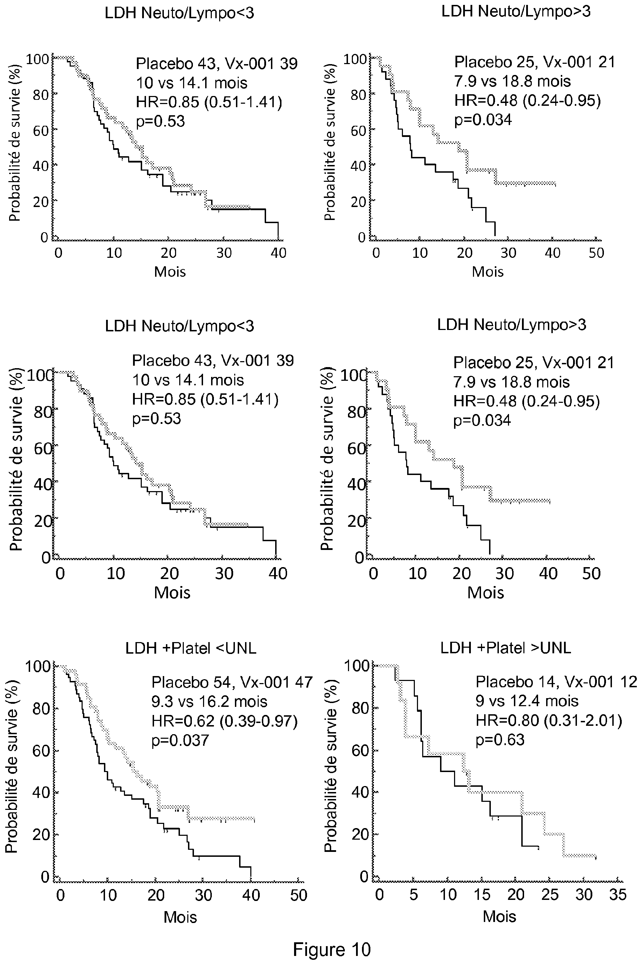

La combinaison des niveaux de LDH et de gGT est la seule combinaison de facteurs dont l'effet est synergique, les autres combinaisons donnent des résultats au plus identiques à l'analyse avec le taux de LDH seul (Figure 10).

### 6. Effet des marqueurs LDH et gGT en fonction de l'immunogénicité de la tumeur

L'immunogénicité d'une tumeur dépend de plusieurs facteurs dont les plus importants sont la présence des TIL (Tumor Infiltrating Lymphocytes) au sein de la tumeur et l'expression de la molécule PD-L1 par les cellules tumorales. Nous avons analysé l'effet du Vx-001 en fonction de ces deux paramètres sur 136 patients dont nous disposions des lames de biopsie. Le kit 22C3 pharmDx a été utilisé pour la détection du PD-L1.

L'expression du PD-L1 a été analysé selon les recommandations du kit d'Agilent qui permet la quantification des cellules tumorales qui expriment le PD-L1 (TPS : *tumor proportional score).* Les tumeurs ont été classifiées en trois groupes i) PD-L1 négative (<1% de cellules marquées ; TPS<1%), ii) PD-L1 positive (1-49% de cellules marquées ; TPS 1-49%) et iii) sur-exprimant PD-L1 (>50% de cellules positives ; TPS>50%). L'infiltration tumorale (*Intratrumoral Site,* IS et *Stromal Site,* SS) par des TIL a été qualifiée en i) absent (score 0), ii) très rare (score 1), iii) faible (score 2), iv) moyenne (score 3) et v) forte (score 4).

Les tumeurs ont été classées PD-L1 négatives si moins de 1% des cellules tumorales exprimaient PD-L1 (TPS ; Tumor Proportional Score ; <1%) et TIL négatives si les TIL dans le core tumoral et le stroma tumoral étaient totalement absents (score 0) ou très rares (score 1). Les résultats individuels sont présentés dans le tableau 2.

**Tableau 2 : scores de TIL et TPS des patients pour lesquels ces données sont disponibles.**

| Patient | Arm | TIL (IS) | TIL (SS) | TPS | Patient | Arm | TIL (IS) | TIL (SS) | TPS |
|---|---|---|---|---|---|---|---|---|---|
| 01-02-004 | V | 0 | 0 | <1% | 04-07-029 | P | 1 | 4 | <1% |
| 01-03-007 | V | 0 | 0 | >50% | 04-09-005 | P | 1 | 4 | <1% |
| 01-03-012 | P | 1 | 4 | <1% | 04-10-007 | P | 1 | 2 | <1% |
| 01-03-018 | V | 0 | 0 | <1% | 04-10-008 | V | 1 | 3 | <1% |
| 01-03-023 | P | 0 | 3 | 1-49% | 04-10-014 | V | 1 | 2 | <1% |
| 01-04-011 | V | 0 | 1 | <1% | 04-10-024 | V | 1 | 4 | <1% |
| 01-05-001 | P | 1 | 2 | <1% | 04-10-031 | P | 0 | 0 | <1% |
| 01-05-011 | V | 1 | 3 | <1% | 04-10-034 | P | 0 | 1 | <1% |
| 01-06-006 | P | 1 | 2 | <1% | 04-12-001 | P | 1 | 2 | <1% |
| 01-08-002 | V | 1 | 2 | 1-49% | 04-12-002 | V | 0 | 0 | <1% |
| 01-08-003 | V | 2 | 3 | 1-49% | 04-12-009 | P | 0 | 1 | <1% |
| 01-08-005 | V | 0 | 2 | <1% | 04-13-003 | P | 0 | 0 | <1% |
| 01-09-002 | V | 1 | 2 | 1-49% | 04-13-004 | V | 0 | 0 | <1% |
| 01-09-006 | P | 0 | 0 | >50% | 04-13-011 | P | 1 | 3 | <1% |
| 01-10-002 | V | 1 | 1 | <1% | 04-13-015 | P | 1 | 3 | 1-49% |
| 01-10-004 | P | 0 | 0 | <1% | 05-01-013 | V | 1 | 1 | <1% |
| 02-03-001 | P | 2 | 2 | 1-49% | 05-03-003 | V | 0 | 0 | <1% |
| 02-06-005 | P | 1 | 2 | <1% | 05-04-012 | V | 0 | 0 | <1% |
| 02-07-004 | V | 0 | 0 | <1% | 05-06-005 | V | 2 | 4 | <1% |
| 02-07-007 | V | 0 | 1 | <1% | 05-07-006 | P | 2 | 4 | 1-49% |
| 03-01-005 | V | 0 | 1 | <1% | 05-09-004 | V | 1 | 1 | <1% |
| 03-01-008 | P | 2 | 2 | <1% | 05-09-006 | P | 0 | 0 | <1% |
| 03-02-001 | P | 2 | 3 | <1% | 05-09-008 | V | 1 | 3 | <1% |
| 03-02-042 | V | 1 | 1 | <1% | 05-11-001 | V | 1 | 3 | <1% |
| 03-03-015 | P | 0 | 0 | <1% | 05-11-007 | P | 1 | 4 | <1% |
| 03-03-019 | P | 1 | 3 | <1% | 07-02-004 | P | 0 | 0 | <1% |
| 03-03-020 | V | 1 | 1 | <1% | 07-03-002 | V | 1 | 2 | <1% |
| 03-03-023 | V | 0 | 0 | <1% | 07-03-004 | P | 0 | 1 | <1% |
| 03-03-025 | P | 1 | 3 | <1% | 07-03-011 | P | 2 | 3 | >50% |
| 03-03-028 | V | 1 | 4 | <1% | 07-03-015 | V | 1 | 2 | 1-49% |
| 03-03-031 | P | 0 | 2 | 1-49% | 07-03-016 | P | 1 | 2 | <1% |
| 03-03-049 | P | 0 | 0 | <1% | 07-03-019 | V | 1 | 2 | <1% |
| 03-03-058 | P | 1 | 2 | <1% | 07-03-021 | P | 1 | 3 | >50% |
| 03-03-062 | P | 0 | 0 | <1% | 07-03-022 | V | 1 | 2 | 1-49% |
| 03-03-065 | V | 0 | 2 | <1% | 07-03-030 | V | 0 | 1 | <1% |
| 03-03-086 | V | 0 | 0 | <1% | 07-03-032 | V | 0 | 0 | <1% |
| 03-04-001 | V | 1 | 2 | <1% | 07-04-006 | V | 1 | 3 | <1% |
| 03-04-014 | P | 1 | 0 | <1% | 07-04-010 | P | 1 | 2 | <1% |
| 03-04-025 | P | 1 | 0 | <1% | 07-04-011 | P | 0 | 1 | >50% |
| 03-04-042 | P | 0 | 1 | <1% | 07-04-020 | V | 1 | 3 | 1-49% |
| 03-05-006 | P | 0 | 3 | 1-49% | 07-04-023 | P | 0 | 0 | 1-49% |
| 03-05-007 | V | 0 | 2 | <1% | 07-05-001 | V | 1 | 3 | <1% |
| 03-06-005 | P | 1 | 3 | 1-49% | 07-06-006 | V | 1 | 3 | <1% |
| 03-06-012 | V | 0 | 1 | <1% | 07-06-016 | V | 0 | 0 | <1% |
| 03-06-016 | P | 0 | 0 | <1% | 07-06-018 | V | 1 | 3 | 1-49% |
| 03-07-001 | V | 1 | 3 | 1-49% | 07-06-020 | V | 1 | 1 | <1% |
| 03-07-007 | V | 0 | 0 | <1% | 07-07-011 | V | 0 | 0 | <1% |
| 03-07-008 | P | 0 | 1 | <1% | 07-08-001 | P | 0 | 0 | <1% |
| 03-07-013 | P | 0 | 1 | <1% | 07-10-002 | P | 1 | 2 | 1-49% |
| 03-07-060 | P | 0 | 0 | <1% | 07-10-005 | V | 0 | 0 | <1% |
| 03-07-066 | V | 0 | 0 | <1% | 07-10-012 | V | 0 | 0 | <1% |
| 03-07-071 | P | 1 | 2 | <1% | 07-10-017 | P | 1 | 2 | 1-49% |
| 03-08-013 | P | 0 | 0 | <1% | 08-01-043 | P | 0 | 0 | <1% |
| 03-08-014 | P | 0 | 1 | <1% | 08-01-046 | P | 0 | 0 | <1% |
| 03-10-001 | V | 2 | 4 | <1% | 08-01-050 | V | 0 | 1 | 1-49% |
| 04-01-023 | P | 1 | 4 | 1-49% | 08-01-051 | P | 0 | 0 | <1% |
| 04-01-057 | V | 0 | 0 | <1% | 08-01-056 | V | 1 | 3 | <1% |
| 04-02-002 | V | 1 | 3 | <1% | 08-01-059 | V | 1 | 3 | <1% |
| 04-03-008 | P | 1 | 3 | <1% | 08-01-060 | V | 1 | 2 | <1% |
| 04-03-010 | V | 1 | 2 | <1% | 08-01-061 | V | 1 | 2 | >50% |
| 04-04-002 | P | 0 | 2 | <1% | 08-01-064 | P | 0 | 1 | <1% |
| 04-05-002 | P | 1 | 2 | 1-49% | 08-01-069 | P | 1 | 3 | <1% |
| 04-05-026 | V | 1 | 3 | <1% | 08-01-070 | V | 0 | 1 | <1% |
| 04-05-036 | V | 0 | 0 | <1% | 08-01-071 | P | 1 | 2 | >50% |
| 04-06-001 | V | 1 | 3 | <1% | 08-01-072 | P | 1 | 3 | <1% |
| 04-06-002 | P | 0 | 0 | <1% | 08-01-073 | P | 0 | 1 | >50% |
| 04-07-013 | P | 1 | 3 | <1% | 08-02-016 | P | 1 | 2 | <1% |
| 04-07-021 | P | 1 | 2 | <1% | 08-03-008 | P | 0 | 1 | >50% |

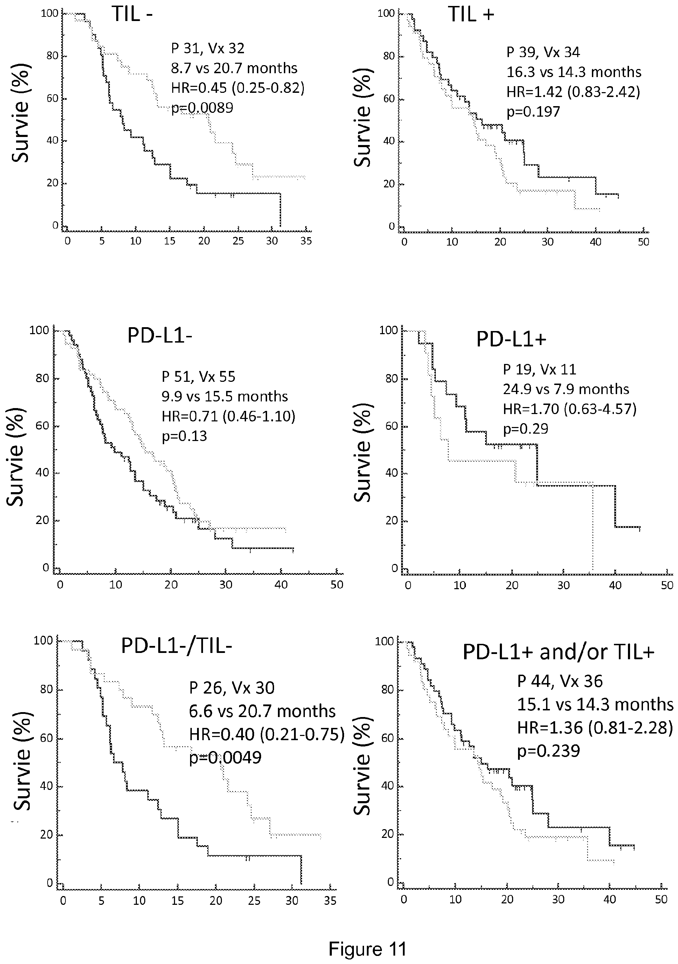

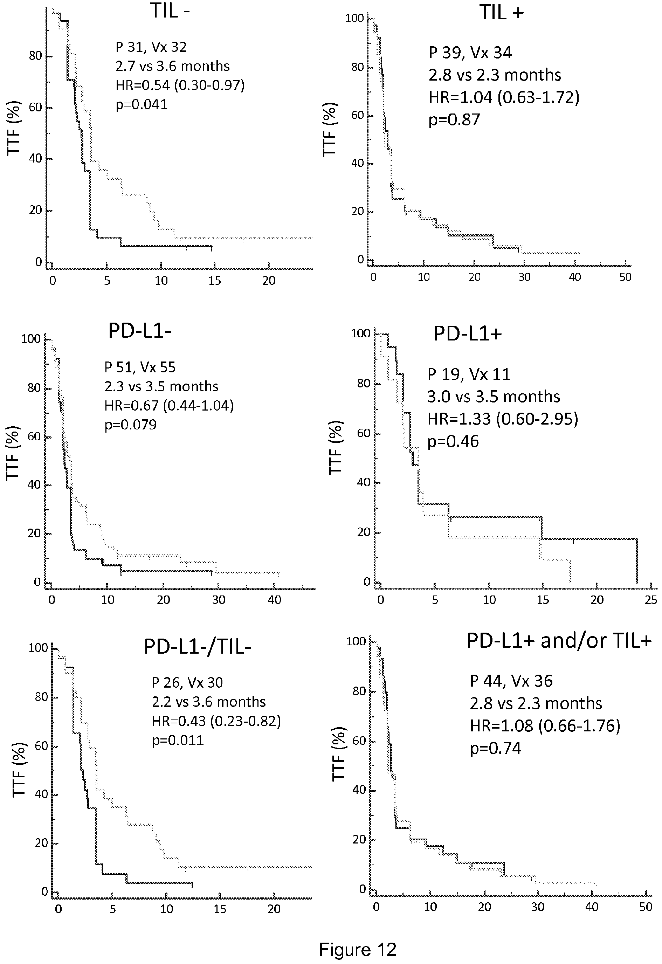

Les résultats présentés sur la figure 11 montrent que le Vx-001 prolonge la survie des patients qui ont des tumeurs négatives pour TIL (8.7 vs 20.7 mois, p=0.0089) et pour PD-L1 (9.9 vs 15,5 mois ; p=0.13) mais ne prolonge pas la survie des patients qui ont des tumeurs positives pour TIL (16,3 vs 14,3 mois ; p=0.197) et pour PD-L1 (24,9 vs 7,9 mois ; p=0,29). L'efficacité clinique du Vx-001 est encore plus importante chez des patients avec des tumeurs qui sont à la fois TIL- et PD-L1- (6,6 vs 20,7 mois ; p=0,0049). Des résultats similaires ont été obtenus avec l'analyse du TTF. Le Vx-001 prolongeait le TTF chez les patients qui avaient des tumeurs TIL- (2,7 vs 3,6 mois ; p=0,041), PD-L1- (2,3 vs 3,5 mois ; p=0,079) et TIL-/PD-L1- (2,2 vs 3,6 mois ; p=0,011) (Figure 12).

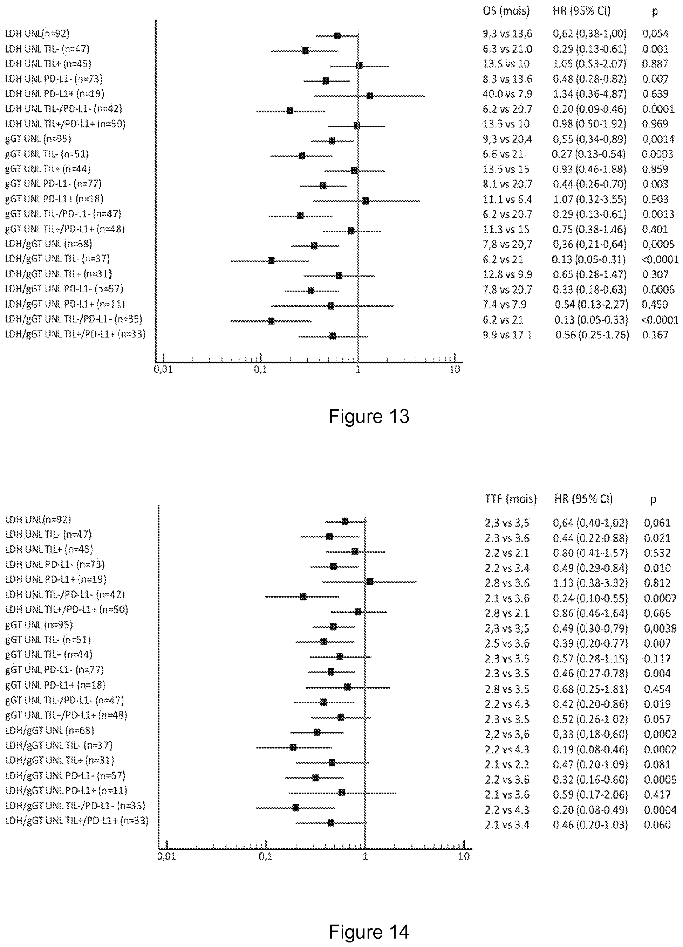

L'analyse de l'effet des deux facteurs LDH et gGT en fonction de l'immunogénicité des tumeurs, définie par la présence des TIL et l'expression du PD-L1, a montré que le taux normal de la LDH sérique et de la gGT sérique corrèle avec une plus longue survie (Figure 13) et un plus long TTF (Figure 14) chez des patients qui ont des tumeurs non-immunogéniques (TIL-, PD-L1-, TIL-/PD-L1-) mais pas chez des patients qui ont des tumeurs immunogéniques (TIL+, PD-L1+, TIL+ et/ou PDI-L1+).

### Références

Ferrucci PF, Ascierto PA, et al., Baseline neutrophils and derived neutrophil-to-lymphocyte ratio: prognostic relevance in metastatic melanoma patients receiving ipilimumab. Ann Oncol. 2016 Apr;27(4):732-8.
Georgoulias V, et al., A multicenter randomized phase IIb efficacy study of Vx-001, a peptide-based cancer vaccine as maintenance treatment in advanced non-small-cell lung cancer: treatmentrationale and protocoldynamics. Clin Lung Cancer. 2013 Jul;14(4):461-5.
Menez-Jamet J, et al., Optimized tumor cryptic peptides: the basis for universal neo-antigen-like tumor vaccines. Ann Transl Med. 2016 Jul;4(14):266.
Mezquita, L. et al., Association of the Lung Immune Prognostic Index With Immune checkpoint Inhibitor Outcomes in Patients With Advanced Non-Small Cell Lung Cancer. JAMA Oncol. 2018 Mar 1;4(3):351-357.Tourdot S, et al., A general strategy to enhance immunogenicity of low-affinity HLA-A2. 1-associated peptides: implication in the identification ofcryptic tumor epitopes. Eur J Immunol. 2000 Dec;30(12):3411-21.

## Revendications

1. Vaccin anticancéreux comprenant le peptide de SEQ ID No : 1 et le peptide de SEQ ID No : 2 (Vx-001), pour son utilisation dans une méthode de traitement d'une tumeur exprimant la télomérase transcriptase inverse (TERT), chez un patient HLA-A*0201, **caractérisé en ce qu'**il est administré à un patient présentant un taux normal de gamma Glutamine Transférase (gGT) et/ou un taux normal lactate déshydrogénase (LDH).

2. Vaccin anticancéreux Vx-001 selon la revendication 1, pour son utilisation selon la revendication 1, **caractérisé en ce qu'**il est administré à un patient présentant un taux normal de gGT et un taux normal de LDH.

3. Vaccin anticancéreux Vx-001 selon la revendication 1, pour son utilisation selon la revendication 1 ou la revendication 2, **caractérisé en ce qu'**il est administré à un patient atteint d'un cancer du poumon non à petites cellules.

4. Vaccin anticancéreux Vx-001 selon la revendication 1, pour son utilisation selon l'une quelconque des revendications 1 à 3, **caractérisé en ce qu'**il est administré à un patient à la suite d'une chimiothérapie de première ligne.

5. Vaccin anticancéreux Vx-001 selon la revendication 1, pour son utilisation selon l'une quelconque des revendications 1 à 4, **caractérisé en ce qu'**il est administré à un patient masculin.

6. Vaccin anticancéreux Vx-001 selon la revendication 1, pour son utilisation selon l'une quelconque des revendications 1 à 5, **caractérisé en ce qu'**il est administré à un patient présentant une tumeur non-épidermoïde.

7. Vaccin anticancéreux Vx-001 selon la revendication 1, pour son utilisation selon l'une quelconque des revendications 1 à 6, **caractérisé en ce qu'**il est administré à un patient de moins de 65 ans.

8. Vaccin anticancéreux Vx-001 selon la revendication 1, pour son utilisation selon l'une quelconque des revendications 1 à 7, **caractérisé en ce qu'**il est administré à un patient dont le cancer ne progresse plus après une chimiothérapie de première ligne.

9. Vaccin anticancéreux Vx-001 selon la revendication 1, pour son utilisation selon l'une quelconque des revendications 1 à 8, **caractérisé en ce qu'**il est administré à un patient ayant un score ECOG 0.

10. Vaccin anticancéreux Vx-001 selon la revendication 1, pour son utilisation selon l'une quelconque des revendications 1 à 9, **caractérisé en ce qu'**il est administré à un patient dont la tumeur n'exprime pas la molécule PD-L1 et/ou n'est pas infiltrée par des lymphocytes.

11. Méthode pour déterminer *in vitro* si un patient HLA-A*0201 atteint d'une tumeur exprimant la TERT est susceptible de répondre favorablement à une vaccination anticancéreuse avec le Vx-001, comprenant une étape de mesure, dans un échantillon biologique dudit patient, du taux de gGT et du taux de LDH dudit patient, **caractérisée en ce que** si au moins un de ces taux est normal, le patient est susceptible de répondre favorablement à la vaccination anticancéreuse.

12. Méthode selon la revendication 11, **caractérisée en ce que** si les taux de gGT et de LDH du patient sont tous les deux normaux, le patient est susceptible de répondre favorablement à la vaccination anticancéreuse par Vx-001.

13. Méthode selon la revendication 11 ou la revendication 12, **caractérisée en ce que** la probabilité que le patient réponde favorablement à la vaccination anticancéreuse par Vx-001 est encore plus élevée si la tumeur n'exprime pas la molécule PD-L1 et/ou n'est pas infiltrée par des lymphocytes.

14. Méthode selon l'une quelconque des revendications 11 à 13, **caractérisée en ce que** la probabilité que le patient réponde favorablement à la vaccination anticancéreuse par Vx-001 est encore plus élevée si le patient présente une ou plusieurs des caractéristiques suivantes :
(i) il est de sexe masculin
(ii) il a une tumeur non-épidermoïde
(iii) il a moins de 65 ans
(iv) il a une tumeur qui ne progresse plus après une première ligne de chimiothérapie
(v) il a un ECOG=0

## Patentansprüche

1. Krebsimpfstoff, umfassend das Peptid von SEQ ID Nr. 1 und das Peptid von SEQ ID Nr. 2 (Vx-001) zur Verwendung in einem Verfahren zur Behandlung eines Tumors, der die Telomerase Reverse Transkriptase (TERT) exprimiert, bei einem HLA-A*0201-Patienten, **dadurch gekennzeichnet, dass** er einem Patienten verabreicht wird, der einen normalen Gamma-Glutamin-Transferase(gGT)-Wert und/oder einen normalen Laktat-Dehydrogenase(LDH)-Wert aufweist.

2. Krebsimpfstoff Vx-001 nach Anspruch 1 für seine Verwendung nach Anspruch 1, **dadurch gekennzeichnet, dass** er einem Patienten verabreicht wird, der einen normalen gGT-Wert und einen normalen LDH-Wert aufweist.

3. Krebsimpfstoff Vx-001 nach Anspruch 1, für seine Verwendung nach Anspruch 1 oder Anspruch 2, **dadurch gekennzeichnet, dass** er einem Patienten verabreicht wird, der an nicht-kleinzelligem Lungenkrebs leidet.

4. Krebsimpfstoff Vx-001 nach Anspruch 1, für seine Verwendung nach einem der Ansprüche 1 bis 3, **dadurch gekennzeichnet, dass** er einem Patienten im Anschluss an eine Erstlinien-Chemotherapie verabreicht wird.

5. Krebsimpfstoff Vx-001 nach Anspruch 1, für seine Verwendung nach einem der Ansprüche 1 bis 4, **dadurch gekennzeichnet, dass** er einem männlichen Patienten verabreicht wird.

6. Krebsimpfstoff Vx-001 nach Anspruch 1, für seine Verwendung nach einem der Ansprüche 1 bis 5, **dadurch gekennzeichnet, dass** er einem Patienten mit einem nicht-epidermalen Tumor verabreicht wird.

7. Krebsimpfstoff Vx-001 nach Anspruch 1, für seine Verwendung nach einem der Ansprüche 1 bis 6, **dadurch gekennzeichnet, dass** er einem Patienten unter 65 Jahren verabreicht wird.

8. Krebsimpfstoff Vx-001 nach Anspruch 1, zur Verwendung nach einem der Ansprüche 1 bis 7, **dadurch gekennzeichnet, dass** er einem Patienten verabreicht wird, dessen Krebs nach der Erstlinien-Chemotherapie nicht mehr fortschreitet.

9. Krebsimpfstoff Vx-001 nach Anspruch 1, für seine Verwendung nach einem der Ansprüche 1 bis 8, **dadurch gekennzeichnet, dass** er einem Patienten mit einem ECOG-Status von 0 verabreicht wird.

10. Krebsimpfstoff Vx-001 nach Anspruch 1, für seine Verwendung nach einem der Ansprüche 1 bis 9, **dadurch gekennzeichnet, dass** er einem Patienten verabreicht wird, dessen Tumor das PD-L1-Molekül nicht exprimiert und/oder nicht durch Lymphozyten infiltriert ist.

11. Verfahren zur In-vitro-Bestimmung, ob ein HLA-A*0201-Patient, der an einem TERT exprimierenden Tumor leidet, wahrscheinlich positiv auf eine Krebsimpfung mit Vx-001 anspricht, umfassend einen Schritt des Messens des gGT-Werts und des LDH-Werts des Patienten in einer biologischen Probe des Patienten, **dadurch gekennzeichnet, dass**, wenn mindestens einer dieser Werte normal ist, der Patient wahrscheinlich positiv auf die Krebsimpfung anspricht.

12. Verfahren nach Anspruch 11, **dadurch gekennzeichnet, dass**, wenn der gGT- und der LDH-Wert des Patienten beide normal sind, der Patient wahrscheinlich positiv auf die Krebsimpfung mit Vx-001 anspricht.

13. Verfahren nach Anspruch 11 oder Anspruch 12, **dadurch gekennzeichnet, dass** die Wahrscheinlichkeit, dass der Patient positiv auf die Krebsimpfung mit Vx-001 anspricht, noch höher ist, wenn der Tumor das PD-L1-Molekül nicht exprimiert und/oder nicht durch Lymphozyten infiltriert ist.

14. Verfahren nach einem der Ansprüche 11 bis 13, **dadurch gekennzeichnet, dass** die Wahrscheinlichkeit, dass der Patient positiv auf die Krebsimpfung mit Vx-001 anspricht, noch höher ist, wenn der Patient eines oder mehrere der folgenden Merkmale aufweist:
(i) er ist männlich
(ii) er hat einen nicht-epidermalen Tumor
(iii) er ist unter 65 Jahre alt
(iv) er hat einen Tumor, der nach einer Erstlinien-Chemotherapie nicht mehr fortschreitet
(v) er hat einen ECOG = 0

## Claims

1. Anti-cancer vaccine comprising the peptide of SEQ ID No. 1 and the peptide of SEQ ID No. 2 (Vx-001), for its use in a method of treating a tumor expressing telomerase reverse transcriptase (TERT), in an HLA-A*0201 patient, **characterized in that** it is administered to a patient having a normal level of gamma Glutamine Transferase (gGT) and/or a normal level of lactate dehydrogenase (LDH).

2. Vx-001 anti-cancer vaccine according to claim 1, for its use according to claim 1, **characterized in that** it is administered to a patient having a normal level of gGT and a normal level of LDH.

3. Vx-001 anti-cancer vaccine according to claim 1, for its use according to claim 1 or claim 2, **characterized in that** it is administered to a patient suffering from a non-small cell lung cancer.

4. Vx-001 anti-cancer vaccine according to claim 1, for its use according to any one of claims 1 to 3, **characterized in that** it is administered to a patient further to a first-line chemotherapy.

5. Vx-001 anti-cancer vaccine according to claim 1, for its use according to any one of claims 1 to 4, **characterized in that** it is administered to a male patient.

6. Vx-001 anti-cancer vaccine according to claim 1, for its use according to any one of claims 1 to 5, **characterized in that** it is administered to a patient having a non-squamous tumor.

7. Vx-001 anti-cancer vaccine according to claim 1, for its use according to any one of claims 1 to 6, **characterized in that** it is administered to a patient less than 65 years old.

8. Vx-001 anti-cancer vaccine according to claim 1, for its use according to any one of claims 1 to 7, **characterized in that** it is administered to a patient whose cancer is no longer progressing after a first-line chemotherapy.

9. Vx-001 anti-cancer vaccine according to claim 1, for its use according to any one of claims 1 to 8, **characterized in that** it is administered to a patient having an ECOG score of 0.

10. Vx-001 anti-cancer vaccine according to claim 1, for its use according to any one of claims 1 to 9, **characterized in that** it is administered to a patient whose tumor does not express the PD-L1 molecule and/or is not infiltrated with lymphocytes.

11. Method for determining in vitro whether an HLA-A*0201 patient suffering from a TERT-expressing tumor is likely to respond favorably to an anti-cancer vaccination with Vx-001, comprising a step of measuring, in a biological sample from said patient, the level of gGT and the level of LDH of said patient, **characterized in that** if at least one of these levels is normal, the patient is likely to respond favorably to the anti-cancer vaccine.

12. Method according to claim 11, **characterized in that** if the patient's levels of gGT and of LDH are both normal, the patient is likely to respond favorably to the anti-cancer vaccination with Vx-001.

13. Method according to claim 11 or claim 12, **characterized in that** the probability of the patient responding favorably to the anti-cancer vaccination with Vx-001 is still higher if the tumor does not express the PD-L1 molecule and/or is not infiltrated with lymphocytes.

14. Method according to any one of claims 11 to 13, **characterized in that** the probability of the patient responding favorably to the anti-cancer vaccination with Vx-001 is still higher if the patient presents one or more of the following characteristics:
(i) he is of the male sex
(ii) he has a non-squamous tumor
(iii) he is less than 65 years old
(iv) he has a tumor which is no longer progressing after a first-line chemotherapy
(v) he has ECOG=0
